# EUROPEAN PATENT SPECIFICATION

(11) **EP 0 593 697 B1**
(45) Date of publication and mention of the grant of the patent: **06.08.1997**
(21) Application number: 93905278.3
(22) Date of filing: 26.02.1993
(51) Int. Cl.: C07D 333/36, C07D 323/00

(54) **MERCAPTO-ACETAMIDE DERIVATIVES**
MERCAPTO-ACETAMID-DERIVATE
DERIVES DE MERCAPTO-ACETAMIDE

(30) Priority: 18.03.1992 IT MI920631
(43) Date of publication of application: 27.04.1994
(73) Proprietor: EDMOND PHARMA S.R.L., I-20151 Milano (IT)
(72) Inventor: NICOLA, Massimo, I-27100 Pavia (IT); INGLESI, Marco, I-20099 Sesto San Giovanni (IT); FREGNAN, Giancarlo, B., I-20156 Milano (IT); VANDONI, Guido, I-20050 Correzzana (IT)
(74) Representative: Dragotti, Gianfranco
(86) International application number: EP9300449
(87) International publication number: WO9319024

(56) References cited:
- EP-A- 0 061 386
- EP-A- 0 192 191
- FR-A- 2 518 542
- FR-A- 2 561 105
- IL FARMACO EDIZIONE SCIENTIFICA vol. 41, no. 10, 1986, Italy, pages 758-780, G. PICCIOLA
- IL FARMACO EDIZIONE SCIENTIFICA vol. 41, no. 1, 1986, Italy, pages 69-79, M. GOBETTI
- CHEMICAL ABSTRACTS vol. 109, no. 3, 1988, Columbus, Ohio, US, abstract no. 17008a, G.B. FREGNAN

## Description

The present invention relates to novel pharmacologically active mercapto-acetamide derivatives, more particularly to derivatives of N-(2-mercaptoacetyl)homocysteine, both in its cyclized γ-thiolactonic form or in the open chain form, to the processes for their preparation and to the pharmaceutical compositions containing them. The new compounds have free-radical scavenging activity preventing cellular damage from toxic metabolites of oxygen such as superoxide radical, hydrogen peroxide and singlet oxygen.

In *"ll Farmaco",* Vol.XLI, n. 1, Jan.1986, pages 69-79, some DL-homocisteine thiolactone derivatives are described, having mucolytic activity, and having general formula: 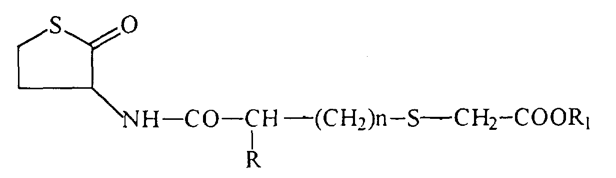 wherein:
R= H, CH₃, C₂H₅, and
R₁= H, K, C₂H₅, Ph, p.NO₂Ph, o.CH₃OPh.

The new compounds also have an in vivo local antiinflammatory activity as well as a mucolytic and mucokinetic activity. They can therefore be useful in the treatment of chronic and acute bronchitis, in the treatment of poisoning and in other pathologies due to the action of toxic free radicals.

More particularly, it is an object of the present invention to provide mercaptoacetamide derivatives of formula (I): 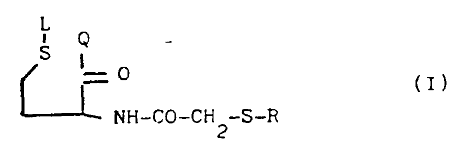 wherein:
- R represents a hydrogen atom, a C₁₋₃ alkyl, a C₇₋₉ aralkyl or a group COR₁ in which R₁ is a C₁₋₃ alkyl or a C₇₋₉ aralkyl, the aralkyl groups being unsubstituted or substituted on the phenyl ring by one or more halogens, C₁₋₃ alkyl or C₁₋₃ alkoxy;
- L is hydrogen
- Q is hydroxy
- or L and Q, together, form a bond, and physiological acceptable solvates, inclusion complexes and, when L is hydrogen and Q is hydroxy, physiological acceptable salts of said mercapto-acetamide derivates.

The compounds of formula (I) may exist as physiologically acceptable solvates, such as hydrates or inclusion complexes, for example with cyclodextrins which constitute a further feature of the present invention. The compounds of formula (I) according to the present invention contain a chiral centre. The present invention includes in its scope both the individual isomers and the mixtures thereof.

The compounds of formula (I) in which L is hydrogen and Q is hydroxyl are carboxylic acids and can exist also as salts with inorganic ions such as sodium, potassium, magnesium or aluminum, with organic ions such as quaternary ammonium compounds, or with organic bases such as, for instance, triethanolamine or tromethamine.

The compounds of general formula (I) of the present invention are prepared by a process which comprises reacting homocysteine- γ -thiolactone of formula (II): 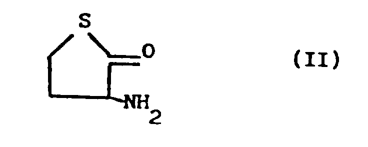 with a functional derivative of mercaptoacetic acid of formula (III):

R-S-CH₂-COOH (III)

wherein R is as defined in claim 1, then when a compound of formula 1 in which L is hydrogen and Q is hydroxyl is to be prepared, subjecting the compound thus obtained to a mild alkaline hydrolysis and optionally converting the compound thus obtained in one of its physiologically acceptable salts.

As functional derivatives of the acids of formula (III) and (IV) any compound capable of reacting with a free amino or mercapto group can be used, such as the acid chloride, the anhydride, a mixed anhydride, an active ester, such as the p-nitrophenylester, or the acid itself, duly activated with, for example, dicyclohexylcarbodiimide.

The reactions are carried out in an aprotic solvent such as toluene, acetonitrile, tetrahydrofurane, methylene chloride, 1,1-dichloroethane, chloroform or dimethylformamide at a temperature ranging from 0° to 100°C, preferably at room temperature, between 20° and 30°C when the action of the amine (II) with the functional derivative of the acid (III) is to be performed, or between 40° and 70°C when the compound of formula (I), wherein R is hydrogen, is reacted with a functional derivative of the acid of formula (IV).

When the acid chloride is used as a functional derivative of the acids of formula (III) or (IV), it is advantageous to carry out the condensation in the presence of an acid acceptor such as sodium hydrogencarbonate or thiethylamine.

The transformation of the cyclic compound thus obtained (formula I, where L and Q, together, form a bond) is carried out by mild hydrolysis in basic conditions using, for example, sodium hydrogen carbonate, sodium or potassium carbonate or sodium or potassium hydroxide. The reaction is carried out in alcohols like methanol, ethanol or isopropanol, or in water or in mixture of water with water miscible solvents like alcohols, acetone, acetonitrile, at temperatures ranging from 0°C to 100°C, preferably from 0°C to 25°C.

Compounds of formula II obtained by this method are in the form of salts: the free acid form can be isolated by conventional methods, such as, for instance, acid-base extraction or ion-exchange resins.

The new compounds of the present invention possess interesting pharmacological properties and are useful as active ingredients for pharmaceutical compositions. More particularly, the mercaptoacetamides of the present invention have shown a good free radical scavenging activity in the test of intoxication by paraquat in mice. Swiss female mice weighing 18-20 g, treated orally with the compounds under examination, are intoxicated, one hour after this administration, with 50 mg/kg of paraquat by intraperitoneal route. The animals are then observed for a week and deaths reported daily. In this test, the compounds of the present invention have shown a good scavenging activity, even superior to that of glutathione.

Moreover, the compounds of the present invention have good antiinflammatory activity. In the test of the carrageenin induced edema in rat paw they are as active as acetylsalicylic acid at the oral doese of 300 mg/kg. The compounds of the present invention also possess a good mucolytic activity as shown in the test of the bronchitis induced in rabbit by exposure to a sulfuric acid aerosol according to Cantorelli et al. - il Farmaco - Ed. Prat. 1979, 34, page 393. More particularly, compounds 1 and 4 to 7, administered, at an oral dose of 300 mg/kg, produced a percent lowering of mucus viscosity significantly greater than that of N-acetylcysteine administered at 400 mg/kg.

The compounds of the present invention have a very low toxicity, largely compatible with their use as medicaments presented in form of pharmaceutical compositions. According to a further feature of the present invention there are provided pharmaceutical compositions comprising as active ingredient at least one compound of formula (I) as hereinbefore defined or a physiologically compatible solvate or salt thereof in association with pharmaceutical carriers or excipients. For pharmaceutical administration the compounds of the present invention may be incorporated into the conventional pharmaceutical preparations in either solid or liquid form. The composition may, for example, be presented in a form suitable for oral, rectal or aerosol administration. Preferred forms include tablets, coated tablets, capsules, sachets containing powder for oral administration, syrups, dry syrups, vials for aerosol. The active ingredient may be incorporated in excipients or carrier conventionally used in pharmaceutical compositions. Each dosage unit may conveniently contain from 50 mg to 1000 mg and preferably to 100 mg to 500 mg of the above ingredient. The following examples illustrate some of the new compounds according to the present invention these examples are not to be in any way considered limitative of the scope of the invention itself.

### EXAMPLE I

### N-(2-mercaptoacetyl)-2-oxo-3-aminotetrahydro tiophene (Compound 1)

To a solution of 15 g homocysteine-tiolactone in tetrahydrofurane (THF) (130 ml) dicyclohexylcarbodiimide (DCC) (26,4 g) and thioglycolic acid (11.8 g) were added. The mixture was stirred for 26 hours at room temperatur. The white precipitate was filtered off, the solvent was evaporated and the residue was purified by column cromatography (silicagel, methylene chloride/Methanol 99:1), obtained 14 g of the desired product.
M.p. 70-72°C

| Analysis C₆H₉NO₂S₂ | | | |
|---|---|---|---|
| Calc.% | C 37.67 | H 4.74 | N 7.32 |
| Found.% | C 37.70 | H 4.71 | N 7.29 |

According to the procedure above described, starting from homocysteine-γ-thiolactone and, respectively, ethylthioacetic acid and benzylthioacetic acid, the following compounds were prepared.

### N-(2-ethylthio) acetyl-2-oxo-3-aminotetrahydrothiophene

### (Compound 2)

### N-(2-benzylthio) acetyl-2-oxo-3-aminotetrahydrothiophene

### (Compound 3)

### EXAMPLE 2

### N-(2-acetyl)thioacetyl-2-oxo-3-aminotetrahydrothiophene

### (Compound 4)

To a solution of compound I (3 g) in THF (20 ml) and triethylamine (2 ml) acetyl chloride (1.4 ml) was added.

The mixture was stirred for 5 min at 0°C. The solvent was evaporated and the residue purified by column chromatography (silicagel, methylene chloride/methanol 98:2) yielding 1.97 g of the title compound.
M.p. 105-108°C

| Analysis C₈H₁1NO₃S₂ | | | |
|---|---|---|---|
| Calc.% | C 41.18 | H 4.75 | N 6.00 |
| Found.% | C 41.13 | H 4.78 | N 5.95 |

According to the procedure above described, the following compounds were prepared:

### N-(2-ethoxycarbonyl) thioacetyl-1-oxo-3-aminotetrahydrothiophene (Compound 5) as an oil.

| Analysis C₈H₁3NO₄S₂ | | | |
|---|---|---|---|
| Calc.% | C 41.05 | H 4.97 | N 5.32 |
| Found.% | C 40.95 | H 5.03 | N 5.30 |

### N-(2-phenylacetyl) thioacetyl-2-oxo-3-aminotetrahydrothiophene (Compound 6).

M.p. 98-100°C

| Analysis C₁4H₁5NO₃S₂ | | | |
|---|---|---|---|
| Calc.% | C 54.34 | H 4.88 | N 4.52 |
| Found.% | C 54.30 | H 4.91 | N 4.48 |

### EXAMPLE 3

### N-(2-mercaptoacetyl) homocysteine, sodium salt (Compound 7)

A suspension of compound 1 (3 g) in 16 ml of IN NaOH was stirred at room temperature until complete solution was obtained. Evaporation of the solvent afforded the title compound.
M.p. 149-155°C

| Analysis C₆H₁0NNaO₃S₂ | | | |
|---|---|---|---|
| Calc.% | C 31.16 | H 4.36 | N 6.05 |
| Found.% | C 30.94 | H 4.40 | N 6.11 |

### EXAMPLE 4

### Tablets

- Compound 1 300 mg
- Lactose 220 mg
- Corn starch 76 mg
- Magnesium stearate 4 mg

Method of preparation: the active ingredient, lactose and corn starch were mixed and homogeneously moistened with water. After screening of the moist mass and drying in a tray dryer, the mixture was again passed through a screen and magnesium stearate was added. The mixture was then pressed into tablets weighing 600 mg each. Each tablet contains 300 mg of active ingredient.

### EXAMPLE 5

### Capsules

- Compound 4 300 mg
- Lactose 98 mg
- Magnesium stearate 2 mg

Method of preparation: the active ingredient was mixed with auxiliary products and the mixture was passed through a screen and mixed homogeneously in a suitable device. The resulting mixture was filled into hard gelatin capsules (400 mg per capsule). Each capsule contains 300 mg of the active ingredient.

### EXAMPLE 6

### Suppositories

- Compound 6 300 mg
- Semisynthetic glycerides of fatty acids 900 mg

Methods of preparation: the semisynthetic glycerides of fatty acids were melted and the active ingredient was added while stirring. After cooling at the suitable temperature the mass was poured into preformed moulds for suppositories. Each suppository weighs 1200 mg and contains 300 mg of active ingredient.

## Claims

1. A compound selected from the group consisting of mercapto-acetamide derivative of formula: 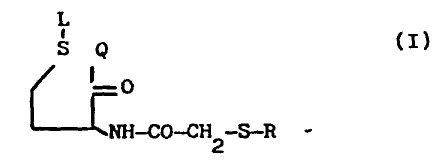 wherein:
- R represents a hydrogen atom, a C₁₋₃ alkyl, a C₇₋₉ aralkyl or a group COR₁ in which R₁ is a C₁₋₃ alkyl or a C₇₋₉ aralkyl, the aralkyl groups being unsubstituted or substituted on the phenyl ring by one or more halogens, C₁₋₃ alkyl or C₁₋₃ alkoxy;
- L is hydrogen
- Q is hydroxy
- or L and Q, together, form a bond, and physiological acceptable solvates, inclusion complexes and, when L is hydrogen and Q is hydroxy, physiological acceptable salts of said mercaptoacetamide derivatives.

2. Physiologically acceptable inclusion complexes of compounds described in Claim 1

3. Physiologically acceptable salts of compounds described in Claim 1, in which L is hydrogen and Q is hydroxy, said salts being formed with sodium, potassium, magnesium or aluminum ions or with triethanolamine or tromethamine.

4. N-(2-mercaptoacetyl)-2-oxo-3-aminotetrahydrothiophene

5. N-(2-acetyl) thioacetyl-2-oxo-3-aminotetrahydrothiophene

6. N-(2-ethoxycarbonyl) thioacetyl-2-oxo-3-aminotetrahydrothiophene

7. N-(2-phenylacetyl) thioacetyl-2-oxo-3-aminotetrahydrothiophene

8. N-(2-mercaptoacetyl) homocysteine and its physiological acceptable salts

9. Compound of Claim 8 as sodium salt

10. Process for the preparation of mercapto-acetamide derivatives according to Claim 1 which comprises reacting homocysteine- -thiolactone of formula 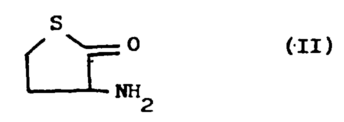 with a functional derivative of mercaptoacetic acid of formula.
R-S-CH₂-COOH (III)
wherein R is as defined in Claim 1 and, when a compound of formula I,
wherein R is hydrogen, is obtained, optionally reacting such a compound with a functional derivative of an acid of formula:
R₁-COOH (IV)
wherein R₁ is as defined in Claim 1 and isolating a mercapto-acetamide derivative of formula (I) wherein R is R₁CO; then, when a compound of formula I in which L is hydrogen and Q is hydroxyl is to be prepared, subjecting the compound thus obtained to a mild alkaline hydrolysis; finally isolating the end product as such or in form of its physiologically acceptable salt, solvate or inclusion product.

11. Process according to Claim 10 characterized in that the hydrolysis is carried out in basic conditions, using sodium hydrogen carbonate, sodium or potassium carbonate or sodium or potassium hydroxide at a temperature between 0°C and 100°C, preferably between 0°C and 25°C.

12. Pharmaceutical compositions comprising as active ingredient at least one compound according to Claims 1 to 9 in association with pharmaceutical carrier or excipients.

13. Pharmaceutical compositions according to Claim 12 as free-radical scavenging agents.

14. Pharmaceutical compositions according to Claim 12 for the treatment of patients suffering from acute or chronic bronchitis, emphysema or intoxicated with substances releasing free radicals.

15. Pharmaceutical compositions according to Claim 12 as local antiinflammatory agents.

16. Pharmaceutical compositions according to Claim 12 as mucolytic or mucokinetic or expectorant agents.

## Patentansprüche

1. Verbindung, ausgewählt aus der Gruppe bestehend aus Mercaptoacetamid-Derivaten der Formel: 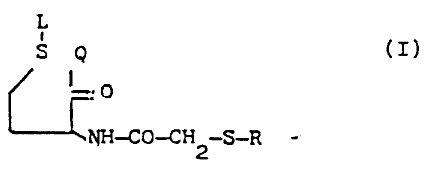 worin R ein Wasserstoffatom, eine C₁₋₃-Alkylgruppe, eine C₇₋₉-Aralkylgruppe oder eine COR₁-Gruppe bedeutet, worin R₁ für eine C₁₋₃-Alkylgruppe oder eine C₇₋₉-Aralkylgruppe steht, wobei die Aralkylgruppen unsubstituiert oder an dem Phenylring durch ein(e) oder mehrere Halogenatome, C₁₋₃-Alkylgruppen oder C₁₋₃-Alkoxygruppen substituiert sind, L Wasserstoff bedeutet, Q für eine Hydroxygruppe steht, oder L und Q gemeinsam eine Bindung bilden; und physiologisch annehmbaren Solvaten, Einschlußkomplexen und, wenn L für Wasserstoff und Q für eine Hydroxygruppe steht, physiologisch annehmbaren Salzen der Mercaptoacetamid-Derivate.

2. Physiologisch annehmbare Einschlußkomplexe der in Anspruch 1 beschriebenen Verbindungen.

3. Physiologisch annehmbare Salze der in Anspruch 1 beschriebenen Verbindungen, worin L für Wasserstoff und Q für eine Hydroxygruppe steht, wobei die Salze mit Natrium-, Kalium-, Magnesium- oder Aluminium-Ionen oder mit Triethanolamin oder Tromethamin gebildet werden.

4. N-(2-Mercaptoacetyl)-2-oxo-3-aminotetrahydrothiophen.

5. N-(2-Acetyl)thioacetyl-2-oxo-3-aminotetrahydrothiophen.

6. N-(2-Ethoxycarbonyl)thioacetyl-2-oxo-3-aminotetrahydrothiophen.

7. N-(2-Phenylacetyl)thioacetyl-2-oxo-3-aminotetrahydrothiophen.

8. N-(2-Mercaptoacetyl)homocystein und dessen physiologisch annehmbare Salze.

9. Verbindung nach Anspruch 8 als Natriumsalz.

10. Verfahren zur Herstellung der Mercaptoacetamid-Derivate nach Anspruch 1, welches das Umsetzen von Homocystein-γ-thiolacton der Formel 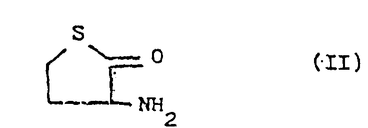 mit einem funktionellen Derivat von Mercaptoessigsäure der Formel
R-S-CH₂-COOH (III),
worin R wie in Anspruch 1 definiert ist, und, wenn eine Verbindung der Formel (I), worin R Wasserstoff bedeutet, erhalten wird, gegebenenfalls das Umsetzen einer derartigen Verbindung mit einem funktionellen Derivat einer Säure der Formel
R₁-COOH (IV),
worin R₁ wie in Anspruch 1 definiert ist, und das Isolieren eines Mercaptoacetamid-Derivats der Formel (I), worin R R₁CO bedeutet; dann, wenn eine Verbindung der Formel (I), worin L für Wasserstoff und Q für eine Hydroxylgruppe steht, hergestellt werden soll, eine schonende alkalische Hydrolyse der so erhaltenen Verbindung; und schließlich das Isolieren des Endprodukts als solches oder in Form seines physiologisch annehmbaren Salzes, Solvates oder Einschlußproduktes umfaßt.

11. Verfahren nach Anspruch 10, dadurch gekennzeichnet, daß die Hydrolyse unter basischen Bedingungen unter Verwendung von Natriumhydrogencarbonat, Natrium- oder Kaliumcarbonat oder Natrium- oder Kaliumhydroxid bei einer Temperatur zwischen 0°C und 100°C, vorzugsweise zwischen 0°C und 25°C, durchgeführt wird.

12. Pharmazeutische Zusammensetzungen, welche als Wirkstoff mindestens eine Verbindung nach den Ansprüchen 1 bis 9 in Verbindung mit pharmazeutischen Trägern oder Exzipienten umfassen.

13. Pharmazeutische Zusammensetzungen nach Anspruch 12 als freie Radikalfänger.

14. Pharmazeutische Zusammensetzungen nach Anspruch 12 zur Behandlung von Patienten, die an akuter oder chronischer Bronchitis, einem Emphysem oder einer Vergiftung mit Stoffen, die freie Radikale freisetzen, leiden.

15. Pharmazeutische Zusammensetzungen nach Anspruch 12 als lokale entzündungshemmende Mittel.

16. Pharmazeutische Zusammensetzungen nach Anspruch 12 als mukolytische oder mukokinetische oder schleimlösende Mittel.

## Revendications

1. Un composé sélectionné dans le groupe constitué de dérivés mercapto-acétamide de formule 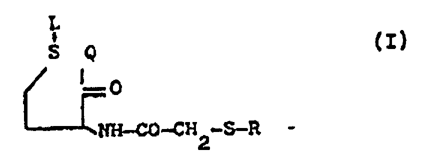 dans laquelle :
- R représente un atome d'hydrogène, un alkyle en C₁₋₃, un aralkyle en C₇₋₉, ou un groupe COR₁ dans lequel R₁ est un alkyle C₁₋₃, ou un aralkyle C₇₋₉, les groupes aralkyles étant non substitués ou substitués sur l'anneau phényle par un ou plusieurs halogènes, un alkyle C₁₋₃ ou un alkoxy C₁₋₃.
- L est l'hydrogène
- Q est un radical hydroxy
- ou L et Q, ensemble, forment une liaison et des solvants physiologiquement acceptables, des complexes d'inclusion et, lorsque L est l'hydrogène et Q un radical hydroxy, des sels physiologiquement acceptables desdits dérivés mercaptoacétamides.

2. Complexes d'inclusion physiologiquement acceptables des composés décrits dans la revendication 1.

3. Sels physiologiquement acceptables des composés décrits dans la revendication 1, dans lesquels L est l'hydrogène et Q est un radical hydroxy, lesdits sels étant formés avec des ions sodium, potassium, magnésium ou aluminium ou avec de la triéthanolamine ou de la trométhamine.

4. N-(2-mercaptoacétyle)-2-oxo-3-aminotétrahydrothiophène

5. N-(2-acétyle)thioacétyle-2-oxo-3-aminotétrahydrothiophène.

6. N-(2-éthoxycarbonyle)thioacétyle-2-oxo-3-aminotétra-hydrothiophène.

7. N-(2-phénylacétyle)thioacétyle-2-oxo-3-aminotétrahydrothiophène.

8. N-(2-mercaptoacétyle) homocystéine et ses sels physiologiques acceptables.

9. Composés selon la revendication 8, sous forme de sel de sodium.

10. Procédé de préparation de dérivés mercapto-acétamides selon la revendication 1, comprenant la mise en réaction d'une homocystéine-thiolactone de formule 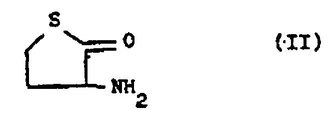 avec un dérivé fonctionnel d'acide mercaptoacétique de formule :
R-S-CH₂-COOH (III)
dans laquelle R est tel que défini dans la revendication 1 et, lorsqu'on obtient un composé de formule I, dans lequel R est l'hydrogène, la mise en réaction en option d'un tel composé avec un dérivé fonctionnel d'un acide de formule :
R₁-COOH (IV)
dans laquelle R₁ est tel que défini dans la revendication 1, et l'isolation d'un dérivé mercapto-acétamide de formule I, dans lequel R est R₁CO_{;} puis, lorsque lorsqu'un composé de formule I, dans lequel L est l'hydrogène et Q un radical hydroxyle doit être préparé, l'exposition du composé ainsi obtenu à une analyse alcaline douce ; enfin isolation du produit final, en tant que tel ou sous la forme de son sel physiologiquement acceptable, d'un solvate ou d'un produit d'inclusion.

11. Produit selon la revendication 10, caractérisé en ce que l'hydrolyse est conduite dans des conditions basiques, par utilisation de carbonate hydrogéné de sodium, de carbonate de sodium ou de potassium ou d'hydroxyde de sodium ou de potassium, à une température comprise dans la plage allant de 0°C à 100°C, de préférence entre 0°C et 25°C.

12. Compositions pharmaceutiques comprenant, à titre d'ingrédient actif, au moins un composé selon les revendications 1 à 9, en association avec un porteur ou des excipients pharmaceutiques.

13. Compositions pharmaceutiques selon la revendication 12, sous forme d'agents de balayage de radicaux libres.

14. Compositions pharmaceutiques selon la revendication 12, pour le traitement de patients souffrant de bronchites aiguës ou chroniques, d'emphysème ou ayant été intoxiqués par des substances dégageant des radicaux libres.

15. Compositions pharmaceutiques selon la revendication 12, à titre d'agents anti-inflammatoires locaux.

16. Compositions pharmaceutiques selon la revendication 12, à titre d'agents mucolytiques ou mucocynétiques ou d'expectorants.
